# EUROPÄISCHE PATENTSCHRIFT

(11) **EP 0 602 108 B1**
(45) Veröffentlichungstag und Bekanntmachung des Hinweises auf die Patenterteilung: **17.01.1996**
(21) Anmeldenummer: 92918426.5
(22) Anmeldetag: 28.08.1992
(51) Int. Cl.: C07C 29/149, C07C 31/20, C07C 33/02

(54) **VERFAHREN ZUR HERSTELLUNG VON UNGESÄTTIGTEN FETTALKOHOLEN**
PROCESS FOR PRODUCING UNSATURATED FATTY ALCOHOLS
PROCEDE DE PRODUCTION D'ALCOOLS GRAS INSATURES

(30) Priorität: 06.09.1991 DE 4129622
(43) Veröffentlichungstag der Anmeldung: 22.06.1994
(73) Patentinhaber: Henkel Kommanditgesellschaft auf Aktien, D-40191 Düsseldorf (DE)
(72) Erfinder: DEMMERING, Günther, D-5650 Solingen (DE)
(86) Internationale Anmeldenummer: EP9201985
(87) Internationale Veröffentlichungsnummer: WO9305003

(56) Entgegenhaltungen:
- EP-A- 0 254 189
- EP-A- 0 280 982
- DE-B- 1 228 603

## Beschreibung

Die Erfindung betrifft ein Verfahren zur Herstellung von ungesättigten Fettalkoholen durch Hydrierung von entsäuerten und/oder nichtentsäuerten Glyceridölen in Gegenwart von Zink-Chrom-Katalysatoren unter weitgehendem Erhalt der im Ausgangsmaterial enthaltenen olefinischen Doppelbindungen und unter Bildung von Propan und 1,2-Propylenglycol.

### Stand der Technik

Aus der Deutschen Patentschrift **DE 25 13 377 B1** ist ein Verfahren zur Herstellung einfach ungesättigter höhermolekularer Fettalkohole durch katalytische Hydrierung freier, ungesättigter, höhermolekularer Fettsäuren oder ihrer mit niedermolekularen, einwertigen Alkoholen gebildeten Ester bekannt. Die Hydrierungsreaktion wird an Stückigen, verhältnismäßig wenig aktiven zinkhaltigen Katalysatoren ohne jeden weiteren Zusatz von niederen Alkoholen als Verdünnungsmittel durchgeführt.

Üblicherweise werden die leicht flüchtigen Fettsäuremethylester von ungesättigten Fettsäuren aus Glyceridölen durch Umesterung mit Methanol erhalten.

Demgemäß hat sich die vorliegende Erfindung zur Aufgabe gestellt, ein Verfahren zur direkten Hydrierung der Triglyceride zur Verfügung zu stellen, bei dem der Umesterungsschritt, nämlich die Bildung von Methylestern aus den Glyceridölen, entfällt.

### Beschreibung der Erfindung

Gegenstand der Erfindung ist ein Verfahren zur Herstellung von ungesättigten Fettalkoholen mit 6 bis 22 C-Atomen, das sich dadurch auszeichnet, daß man entsäuerte und/oder nichtentsäuerte Glyceridöle, die einfach und/oder mehrfach ungesättigte, überwiegend geradkettige und/oder verzweigtkettige Fettsäuren mit 6 bis 22 C-Atomen im Fettsäurerest enthalten, unter Anwendung eines 30- bis 100fachen Überschusses an Wasserstoff im Druckbereich von 50 bis 300 bar bei Temperaturen von 200 bis 350 °C an Zink-Chrom-Katalysatoren vom Spinelltyp unter weitgehendem Erhalt der olefinischen Doppelbindungen direkt und unter Bildung von Propan und 1,2-Propylenglycol hydriert.

Handelsübliche Katalysatoren für die Hydrierung von ungesättigten Fettsäuremethylestern zu ungesättigten Fettalkoholen eignen sich nur in Ausnahmefällen auch für die Hydrierung von nichtentsäuerten Glyceridölen. Überraschenderweise wurde jedoch gefunden, daß die im Sinne der Erfindung zu verwendenden Zink-Chrom-Katalysatoren auch in der Hydrierung von Glyceridölen mit Säurezahlen im Bereich von 0 bis 10 eine ausgezeichnete Aktivität aufweisen. Gemäß der vorliegenden Erfindung kann durch die Wahl der Reaktionstemperatur und der Katalysatorzuschläge die Hydrierung so gelenkt werden, daß sich praktisch kein Glycerin und nur wenig 1,2-Propandiol bildet, sondern als Spaltprodukt bei der Fettalkoholherstellung überwiegend Propan entsteht.

Der Katalysator wird in stückiger Form, d.h. als Tablette, Strangpreßling, Granulat oder Extrudat eingesetzt.

Eine bevorzugte Ausführungsform der vorliegenden Erfindung besteht darin, daß man Glyceridöle verwendet, die aus Olivenöl, erucasäurearmem und erucasäurereichem Rüböl, Fischöl, Rindertalg und/oder Schweineschmalz erhalten werden.

Die Selektivität der Reaktion in Richtung Alkoholbildung und auch in Richtung Erhalt der Doppelbindungen sowie der cis-Konfiguration des Ausgangsöles ist bei dem Verfahren der vorliegenden Erfindung sehr hoch. Die Selektivität des Katalysators kann durch geeignete zusätzliche Dotierungsmittel und Zusätze gesteigert werden.

Eine Ausführungsform der vorliegenden Erfindung besteht darin, daß man Zink-Chrom-Katalysatoren einsetzt, die mit Aluminium, Mangan, Cadmium und/oder Vanadium dotiert sind. Gemäß einer bevorzugten Ausführungsform werden Zink-Chrom-Katalysatoren verwendet, die 1 bis 8, vorzugsweise 1,5 bis 5 Gew.-% der oben genannten Dotierungsmittel - bezogen auf die Katalysatormasse - enthalten. Durch diese Maßnahme kann die Selektivität des Katalysators weiter gesteigert werden.

Eine weitere bevorzugte Ausführungsform besteht darin, daß man Zink-Chrom-Katalysatoren verwendet, die zusätzlich mit Barium- und Alkalimetallzusätzen dotiert sind. Erfindungsgemäß werden Zink-Chrom-Katalysatoren eingesetzt, die 0,1 bis 3, vorzugsweise 0,5 bis 2 Gew.-% an Barium und/oder Alkalimetallen, insbesondere Kalium - bezogen auf die Katalysatormasse - enthalten. Die hohe Standzeit des Katalysators wird durch die Zusätze von Barium und kleinen Alkalimengen weiter verbessert.

Erfindungsgemäß verwendete Katalysatoren können neben den oben genannten Bestandteilen noch übliche Bindemittel enthalten. Eine weitere Ausführungsform der vorliegenden Erfindung besteht darin, den erfindungsgemäß verwendeten Katalysatoren 1 bis 4 Gew.-% an Graphit zur verbesserten Verarbeitbarkeit der Granulate und/oder Extrudate zuzusetzen.

Die folgenden Beispiele sollen den Gegenstand der Erfindung näher erläutern, ohne ihn darauf einzuschränken.

### Beispiele

### Beispiel 1

In einem Kneter wurden 50 kg pulverförmiges Zinkoxid mit 5 l Wasser unter ständigem Kneten mit einer Lösung von 35 kg Chromtrioxid in 30 l Wasser und 2 kg 50 %-iger Kaliumhydroxidlösung gemischt. Die entstandene Masse wurde mit 3 % Graphit versetzt, getrocknet und zu Tabletten von 6 mm Durchmesser und 4 mm Höhe verpreßt.

1,5 l der erhaltenen Katalysatortabletten wurden in den Rohrreaktor (Durchmesser 42 mm; Länge 1500 mm) einer kontinuierlich zu betreibenden Hydrierapparatur gefüllt. Danach wurde der Katalysator mit Wasserstoff reduziert, wobei die Temperatur nach und nach von 180 auf 400°C gesteigert wurde.

Im Anschluß an die Reduktion wurde erucasäurearmes Rüböl (Verseifungszahl 190; Iodzahl 114; Säurezahl 4) mit einer Geschwindigkeit von 300 ml pro Stunde in die Hydrierapparatur eingespeist und bei einer Reaktionstemperatur von 290°C bei 200 bar mit Wasserstoff hydriert. Das rohe Reaktionsprodukt enthielt 2 Gew.-% 1,2-Propylenglycol. Nach dem Abtrennen des Reaktionswassers und des gebildeten 1,2-Propylenglycols wies das erhaltene Fettalkoholgemisch die folgenden Kennzahlen auf:
- Verseifungszahl (VZ) :: 206
- Iodzahl (IZ) :: 180
- Säurezahl (SZ) :: 0,1

In der nachfolgenden Tab.1 ist die Zusammensetzung des Fettsäureanteils in dem als Ausgangsmaterial verwendeten Rüböl (bestimmt am Rübölmethylester) der Zusammensetzung des erhaltenen Fettalkoholgemisches gegenübergestellt.

**Tab. 1:**

| Zusammensetzung Rüböl und Fettalkoholgemisch | | |
|---|---|---|
| Kettenlänge | Rüböl | Fettalkohol |
| | Gew.-% | Gew.-% |
| C 16 gesättigt | 4,6 | 4,7 |
| C 18 gesättigt | 1,5 | 3,8 |
| C 18 einfach ungesättigt | 60,0 | 70,1 |
| C 18 zweifach ungesättigt | 21,0 | 11,5 |
| C 18 dreifach ungesättigt | 9,5 | 6,3 |
| C 20 gesättigt | 3,4 | 3,3 |
| Kohlenwasserstoffe | - | 0,4 |

### Beispiel 2

Unter den in Beispiel 1 genannten Bedingungen und unter Einsatz des dort beschriebenen Katalysator wurde Olivenöl (VZ 180; JZ 85,6; SZ 6,7; 94 Gew.-% cis-Anteile; 6 Gew.-% trans-Anteile) hydriert. Das nach dem Abtrennen des Reaktionswassers und Propandiols verbleibende Fettalkoholgemisch hatte die Kennzahlen VZ 0,3; JZ 87; SZ 0,04; 90 % cis-Anteile; 10 % trans-Anteile.

In der nachfolgenden Tab.2 ist die Zusammensetzung des Fettsäureanteils des als Ausgangsmaterial verwendeten Olivenöls sowie die Zusammensetzung des daraus erhaltenen Fettalkoholgemisches wiedergegeben.

**Tab. 2:**

| Zusammensetzung Olivenöl und Fettalkoholgemisch | | |
|---|---|---|
| Kettenlänge | Olivenöl | Fettalkohol |
| | Gew.-% | Gew.-% |
| C 16 gesättigt | 16,3 | 16,0 |
| C 16 einfach ungesättigt | 1,7 | 1,4 |
| C 18 gesättigt | 2,3 | 3,3 |
| C 18 einfach ungesättigt | 61,8 | 67,0 |
| C 18 zweifach ungesättigt | 16,5 | 10,8 |
| C18 dreifach ungesättigt | 0,7 | 0,4 |
| C 20 gesättigt | 0,7 | 0,6 |
| Kohlenwasserstoffe | - | 0,5 |

### Beispiel 3

In dem Rohrreaktor (Durchmesser 42 mm; Länge 6 000 mm) einer kontinuierlich zu betreibenden Hydrierapparatur wurden 8 l der in Beispiel 1 beschriebenen Katalysatortabletten zunächst bei 180°C und anschließend bei 400°C mit Wasserstoff reduziert. Über den reduzierten Katalysator wurde bei 250 bar und 310°C im Gleichstrom Wasserstoff und erucasäurearmes Rüböl (SZ 0,6; VZ 191; JZ 111) geleitet. Das Öl wurde mit einer Geschwindigkeit von 2 l pro Stunde, entsprechend einer lhsv von 2 l⁻¹h⁻¹ eingespeist. Nach dem Abtrennen des Reaktionswassers und des gebildeten 1,2-Propylenglycols wies das erhaltene Fettalkoholgemisch die Kennzahlen: SZ 0,05; VZ 1,0; JZ 105; OHZ 208 auf.

In der nachfolgenden Tab.3 ist die Zusammensetzung des Fettsäureanteils in dem als Ausgangsmaterial verwendeten erucasäurearmen Rüböl (bestimmt am Rübölmethylester) der Zusammensetzung des erhaltenen Fettalkoholgemisches gegenübergestellt.

**Tab. 3:**

| Zusammensetzung Rüböl und Fettalkoholgemisch | | |
|---|---|---|
| Kettenlänge | Rüböl | Fettalkohol |
| | Gew.-% | Gew.-% |
| C 18 gesättigt | 4,9 | 5,0 |
| C 18 gesättigt | 1,5 | 3,5 |
| C 18 einfach ungesättigt | 60,0 | 64,5 |
| C 18 zweifach ungesättigt | 20,8 | 17,0 |
| C 18 dreifach ungesättigt | 9,3 | 6,0 |
| C 18 gesättigt | 3,4 | 3,5 |
| Kohlenwasserstoffe | - | 0,5 |

### Beispiel 4

Unter den in Beispiel 3 beschriebenen Bedingungen wurde erucasäurereiches Rüböl (SZ 7,8; VZ 170; JZ 102) zu einem Fettalkoholgemisch mit den Kennzahlen SZ 0,02; VZ 1,0; JZ 99; OHZ 182 hydriert.

In der nachfolgenden Tab.4.ist die Zusammensetzung des Fettsäureanteils in dem als Ausgangsmaterial benutzten Rüböl und die Zusammensetzung des daraus erhaltenen Fettalkoholgemisches aufgeführt.

**Tab. 4:**

| Zusammensetzung Rüböl und Fettalkoholgemisch | | |
|---|---|---|
| Kettenlänge | Rüböl | Fettalkohol |
| | Gew.-% | Gew.-% |
| C 18 gesättigt | 3,7 | 3,8 |
| C 18 gesättigt | 0,9 | 3,6 |
| C 18 einfach ungesättigt | 12,9 | 15,2 |
| C 18 zweifach ungesättigt | 14,1 | 10,8 |
| C 18 dreifach ungesättigt | 8,9 | 6,7 |
| C 18 gesättigt | 12,1 | 12,4 |
| C 22 ungesättigt | 47,4 | 50,8 |
| Kohlenwasserstoffe | - | 0,3 |

### Beispiel 5

Unter den in Beispiel 3 beschriebenen Bedingungen wurde Palmöl (SZ 7,4; VZ 194; JZ 50,6) zu einem Fettalkoholgemisch mit den Kennzahlen SZ 0,1; VZ 1,0; JZ 50,4; OHZ 214 hydriert.

In der nachfolgenden Tab.5 ist die Zusammensetzung des Fettsäureanteils in dem als Ausgangsmaterial verwendeten Palmöl (bestimmt am Palmölmethylester) der Zusammensetzung des erhaltenen Fettalkoholgemisches gegenübergestellt.

**Tab. 5:**

| Zusammensetzung Palmöl und Fettalkoholgemisch | | |
|---|---|---|
| Kettenlänge | Palmöl | Fettalkohol |
| | Gew.-% | Gew.-% |
| C 16 | 3,0 | 3,0 |
| C 16 | 45,4 | 45,8 |
| C 18 gesättigt | 4,1 | 5,2 |
| C 18 einfach ungesättigt | 38,0 | 38,8 |
| C 18 zweifach ungesättigt | 8,4 | 6,3 |
| C 18 dreifach ungesättigt | 0,3 | 0,3 |
| C 20 | 0,7 | 0,8 |

### Beispiel 6

Unter den im Beispiel 3 beschriebenen Bedingungen wurde Schweineschmalz (SZ 0,6; VZ 192,3; JZ 60,2) zu einem Fettalkoholgemisch mit den Kennzahlen SZ 0,05; VZ 1,0; JZ 60,4; OHZ 211 hydriert.

In der nachfolgenden Tab.6 ist die Zusammensetzung des Fettsäureanteils in dem als Ausgangsmaterial verwendeten Schweineschmalz (bestimmt am Methylester nach Umesterung) der Zusammensetzung des erhaltenen Fettalkoholgemisches gegenübergestellt.

**Tab. 6:**

| Zusammensetzung Schweineschmalz und Fettalkoholgemisch | | |
|---|---|---|
| Kettenläge | Schweineschmalz | Fettalkohol |
| | Gew.-% | Gew.-% |
| C 16 gesättigt | 1,7 | 1,8 |
| C 16 gesättigt | 25,1 | 26,8 |
| C 16 einfach ungesättigt | 2,3 | 1,9 |
| C 18 gesättigt | 15,6 | 17,5 |
| C 18 einfach ungesättigt | 43,2 | 45,8 |
| C 18 zweifach ungesättigt | 8,7 | 3,7 |
| C 18 dreifach ungesättigt | 0,7 | 0,6 |
| C 18 gesättigt | 2,5 | 2,4 |
| Kohlenwasserstoffe | - | 1,1 |

In den Beispielen 3 bis 6 wurden etwa 80 % der theoretisch zu erwartenden 1,2-Propylenglycolmenge zu Propan hydriert.

Die Katalysatorabschreibung bezogen auf den Öldurchsatz betrug 0,1 %. Vergleichbare Ergebnisse wurden erhalten, wenn Talgöl und Butterfett unter den Bedingungen des Beispiels 3 hydriert wurden.

## Patentansprüche

1. Verfahren zur Herstellung von ungesättigten Fettalkoholen, **dadurch gekennzeichnet**, daß man entsäuerte und/ oder nichtentsäuerte Glyceridöle, die einfach und/oder mehrfach ungesättigte, überwiegend geradkettige und/ oder verzweigtkettige Fettsäuren mit 6 bis 22 C-Atomen im Fettsäurerest enthalten, unter Anwendung eines 30-bis 100fachen Überschusses an Wasserstoff im Druckbereich von 50 bis 300 bar bei Temperaturen von 200 bis 400°C an Zink-Chrom-Katalysatoren vom Spinelltyp unter weitgehendem Erhalt der olefinischen Doppelbindungen und unter Bildung von Propan und 1,2-Propylenglycol direkt hydriert.

2. Verfahren nach Anspruch 1, **dadurch gekennzeichnet**, daß man Glyceridöle hydriert, die aus Olivenöl, erucasäurearmem und erucasäurereichem Rüböl, Fischöl, Rindertalg und/oder Schweineschmalz erhalten werden.

3. Verfahren nach Ansprüchen 1 und 2, **dadurch gekennzeichnet** , daß man Zink-Chrom-Katalysatoren einsetzt, die mit Aluminium, Cadmium und oder Vanadium dotiert sind.

4. Verfahren nach Anspruch 3, **dadurch gekennzeichnet,** daß man Zink-Chrom-Katalysatoren einsetzt, die 1 bis 8 Gew.-% Aluminium, Mangan, Cadmium und/oder Vanadium - bezogen auf die Katalysatormasse - enthalten.

5. Verfahren nach Ansprüchen 3 und 4, **dadurch gekennzeichnet** , daß man Zink-Chrom-Katalysatoren einsetzt, die mit Barium- und Alkalimetallzusätzen dotiert sind.

6. Verfahren nach Anspruch 5, **dadurch gekennzeichnet,** daß man Zink-Chrom-Katalysatoren einsetzt, die 0,1 bis 3 Gew.-% Barium und/oder Alkalimetalle - bezogen auf die Katalysatormasse - enthalten.

7. Verfahren nach mindestens einem der Ansprüche 3 bis 6, **dadurch gekennzeichnet**, daß man Zink-Chrom-Katalyatoren einsetzt, die 1 bis 4 Gew.-% Graphit - bezogen auf die Katalysatormasse - enthalten.

## Claims

1. A process for the production of unsaturated fatty alcohols, **characterized in that** deacidified and/or non-deacidified glyceride oils containing monounsaturated and/or polyunsaturated, predominantly linear and/or branched fatty acids containing 6 to 22 carbon atoms in the fatty acid component are directly hydrogenated with a 30- to 100-fold excess of hydrogen on zinc-chromium catalysts of the spinel type under pressures of 50 to 300 bar and at temperatures of 200 to 400°C with formation of propane and 1,2-propylene glycol, the olefinic bonds remaining largely intact.

2. A process as claimed in claim 1, **characterized in** **that** glyceride oils obtained from olive oil, rapeseed oils poor and rich in erucic acid, fish oil, beef tallow and/or lard are hydrogenated.

3. A process as claimed in claims 1 and 2, **characterized** **in that** zinc-chromium catalysts doped with aluminium, cadmium and/or vanadium are used.

4. A process as claimed in claim 3, **characterized in** **that** zinc-chromium catalysts containing 1 to 8% by weight of aluminium, manganese, cadmium and/or vanadium, based on the weight of the catalyst, are used.

5. A process as claimed in claims 3 and 4, **characterized** **in that** zinc-chromium catalysts doped with additions of barium and alkali metals are used.

6. A process as claimed in claim 5, **characterized in** **that** zinc-chromium catalysts containing 0.1 to 3% by weight of barium and/or alkali metals, based on the weight of the catalyst, are used.

7. A process as claimed in at least one of claims 3 to 6, **characterized in that** zinc-chromium catalysts containing 1 to 4% by weight of graphite, based on the weight of the catalyst, are used.

## Revendications

1. Procédé de fabrication d'alcools gras insaturés, **caractérisé en ce que** l'on hydrogène directement des huiles glycéridiques désacidifiées et/ou non désacidifiées, qui renferment des acides gras mono- et/ou poly-insaturés, en majeure partie à chaîne droite et/ou ramifiée, dont le radical acide gras comporte 6 à 22 atomes de C, en appliquant un excès d'hydrogène de 30 à 100 fois, dans le domaine de pression de 50 à 300 bars, à des températures de 200 à 400 °C, sur des catalyseurs au zinc-chrome du type spinelle, en conservant dans une large mesure les doubles liaisons oléfiniques et sous formation de propane et de 1,2-propylèneglycol.

2. Procédé selon la revendication 1, **caractérisé en** **ce que** l'on hydrogène des huiles glycéridiques, qui sont obtenues à partir d'huile d'olive, d'huile de navette pauvre et riche en acide érucique, d'huile de poisson, de suif de boeuf et/ou de saindoux

3. Procédé selon les revendications 1 et 2, **caractérisé en ce que** l'on met en oeuvre des catalyseurs au zinc-chrome, qui sont dopés avec de l'aluminium, du cadmium et/ou du vanadium.

4. Procédé selon la revendication 3, **caractérisé en** **ce que** l'on met en oeuvre des catalyseurs au zinc-chrome, qui contiennent 1 à 8 % en poids (par rapport à la masse du catalyseur) d'aluminium, de manganèse, de cadmium et/ou de vanadium.

5. Procédé selon les revendications 3 et 4, **caractérisé en ce que** l'on met en oeuvre des catalyseurs au zinc-chrome, qui sont dopés avec des additifs de baryum et de métaux alcalins.

6. Procédé selon la revendication 5, **caractérisé en** **ce que** l'on met en oeuvre des catalyseurs au zinc-chrome, qui contiennent 0,1 à 3 % en poids (par rapport à la masse du catalyseur) de baryum et/ou de métaux alcalins.

7. Procédé selon au moins une des revendications 3 à 6, **caractérisé en ce que** l'on met en oeuvre des catalyseurs au zinc-chrome, qui contiennent 1 à 4 % en poids (par rapport à la masse du catalyseur) de graphite.
